# EUROPEAN PATENT APPLICATION

(11) **EP 4 166 178 A1**
(43) Date of publication of application: **19.04.2023**
(21) Application number: 21202963.1
(22) Date of filing: 15.10.2021
(51) Int. Cl.: A61M 16/04, A61M 25/01, A61B 17/34

(54) **TRACHEOTOMY APPARATUS AND TRACHEOSTOMY SYSTEM**

(71) Applicant: innotrach ApS, 2770 Kastrup (DK)
(72) Inventor: HOFMAN ROSENKRANZ, Søren, 2300 København S (DK)
(74) Representative: Brann AB

(57) **Abstract**

A tracheotomy apparatus (1) and a tracheostomy system are proposed. The tracheotomy apparatus (1) comprises: a knife assembly (30) configured to be in a first state or in a second state. The knife assembly (30) further comprises a knife support (40), and a knife (10) supported by the knife support. The knife has a proximal (12) portion and a distal (14) portion, wherein the distal portion (14) is elongated, and has a distal (20) end, and extends from the knife support (40) in a distal direction. The proximal portion (12) is attached to the knife support (40). The distal portion (14) comprises a cutting edge (24) at the distal end in first the state, and an opening (26) at the distal end in the second state. The distal portion is configured to receive a tracheostomy device extending through the opening (26). The tracheostomy system comprises a tracheotomy apparatus (1) and tracheostomy device.

## Description

### Technical field

The proposed technology relates generally to the field of devices for tracheostomy. Further, the proposed technology relates specifically to the field of tracheotomy apparatuses. The technology also relates to a tracheostomy system comprising a tracheotomy apparatus and tracheostomy device.

### Background

A tracheostomy is a medical procedure ― either temporary or permanent ― that involves creating an opening in the throat in order to place a tube into a person's windpipe.

The tube is inserted through a cut in the throat below the vocal cords. This allows air to enter the lungs. Breathing is then done through the tube, bypassing the mouth, nose, and throat.

A tracheostomy is performed for several reasons, all involving restricted airways. Breathing is done through the tracheostomy tube rather than through the nose and mouth. There are many reasons why sufficient air cannot get to the lungs. It may be done during an emergency when the airway is blocked. It could also be used when a disease or other problem makes normal breathing impossible or difficult. Thus, tracheostomies are performed due to a lack of air getting to the lungs.

A tracheostomy is usually done for one of three reasons: to bypass an obstructed upper airway; to clean and remove secretions from the airway; to more easily, and usually more safely, deliver oxygen to the lungs.

Conditions that may require a tracheostomy include but are note restricted to: anaphylaxis, congenital defects of the airway, burns of the airway from inhalation of corrosive material, cancer, chronic lung disease, coma, diaphragm dysfunction, facial burns or surgery, infection, injury to the larynx, laryngectomy, injury to the chest wall, need for prolonged respiratory or ventilator support, obstruction of the airway by a foreign body, obstructive sleep apnea, paralysis of the muscles used in swallowing severe throat or mouth injuries, tumours, vocal cord paralysis.

Tracheotomy and tracheostomy are two parts of the same surgical procedure which is done to facilitate breathing in a patient who is having issues related to respiration usually following trauma, foreign bodies, neurological problem, etc. The main difference between tracheotomy and tracheostomy is that tracheotomy is a surgical procedure which involves creating a direct airway incision made on the trachea whereas tracheostomy refers to an insertion of a tube through this airway. Additionally, the term "tracheotomy" refers to the incision into the trachea (windpipe) that forms a temporary or permanent opening, which is called a "tracheostomy". However, the terms are sometimes used interchangeably. A tracheostomy, the hole in the throat that the tube passes through, is commonly referred to as a "stoma".

In short, the procedure is usually performed as following. The patient is placed supine so that the throat is fully extended. The throat is palpated, and the anatomy is identified. Sometimes labelling the landmarks with a permanent marker is performed. The ideal location of the tracheostomy is regarded being between the 1st and 3rd tracheal ring. Once the appropriate location is identified, a horizontal skin incision is made, about 2-3 cm in length. The pretracheal tissue is cleared by blunt dissection with a mosquito, or Kelly clamp, until the trachea is clearly palpable. The introducer needle is then used to puncture the anterior wall of the trachea and guide a catheter into the trachea. Further, the introducer needle is withdrawn leaving the catheter in place. A guidewire is fed through the catheter. The catheter is removed over the wire leaving it in place. A dilator, or dilatator, is then used to dilate the track. A tracheostomy tube with inner trocar is then cannulated into the trachea. The tracheostomy tube trocar and wire can then be removed. The tracheostomy tube is secured to the skin with sutures and tracheostomy tape.

The conventional way of providing tracheostomy has several disadvantages. First, the procedure requires several steps, wherein each step involves usage of a separate instrument. That requires well-functioning logistic chain, which includes ordering, transportation, and storage of the medical equipment, due to the requirement of that all the parts must be simultaneously available for performing the procedure.

Moreover, as the parts are usually sterile packaged, there is an expiring date of the each of the packaging. Having several packaging with different expiring dates makes it difficult to overview and maintain the stock.

Further, performing different steps that involves many parts, usually simultaneously to using a bronchoscope, requires several people assisting the surgeon. The more steps are required, the longer the duration of the procedure and therefore, bleeding caused by the procedure.

Another disadvantage is that the length of skin incision may vary depending on the surgeon, and from occasion to occasion. An inappropriate skin incision may result in increasing bleeding risk, infection risk, and complicate recovery and scarring.

### Summary

The proposed technology aims at obviating the aforementioned disadvantages and failings of previously known tracheotomy apparatuses, and at providing an improved tracheotomy apparatus and a tracheostomy system that allows for a straight-forward and robust technology and which is capable of providing a tracheotomy in a short time and improved manner.

A primary object of the proposed technology is to provide an improved tracheotomy apparatus. It is a further object to decrease the tracheotomy procedure duration. Another object is to provide a complete all-in-one system comprising the equipment for performing a tracheotomy. Still another object is to provide a tracheotomy apparatus that is comfortable and easy to use, and preferably requires fewer assisting personnel. In particular, it is an object to provide a tracheotomy apparatus that can be used by a single person.

It is a further object to provide a tracheotomy apparatus that provides a uniform, or standardized, tracheostomy, or stoma, regardless of user. Another object of the proposed technology is to provide an improved tracheostomy system comprising a tracheotomy apparatus and a tracheostomy device.

According to the proposed technology at least the primary object is attained by means of the initially defined tracheotomy apparatus and tracheostomy system having the features defined in the independent claims. Preferred alternative solutions of the proposed technology are further defined in the dependent claims.

According to a first aspect of the proposed technology, there is provided a tracheotomy apparatus of the initially defined type. According to a second aspect of the proposed technology, there is provided a tracheostomy system comprising a tracheotomy apparatus and tracheostomy device.

In a first aspect of the proposed technology, a tracheotomy apparatus is provided. The tracheotomy apparatus comprises:
a knife assembly configured to be in a first, or closed, state or in a second, or open, state. The knife assembly further comprises a knife support, and a knife supported by the knife support. The knife has
a proximal, or rear, portion and a distal, or forward, portion, wherein the distal portion is elongated, and has a distal, or forward, end, and extends from the knife support in a distal direction. The proximal portion is attached to the knife support. The distal portion comprises, or forms, a cutting edge at the distal end in the first state, and an opening at the distal end in the second state. The distal portion is configured to receive a tracheostomy device, such as tracheotomy tube or cannula, extending through the opening.

According to a second aspect of the proposed technology, there is provided a tracheostomy system comprising a tracheotomy apparatus and tracheostomy device, such as a tube, a needle, a surgical instrument, or a catheter.

The tracheostomy system may be used as follows. A user grips the knife assembly, being in a first state. Preferably, the user grips, or supports, the knife support. The user positions the cutting edge at an appropriate placement at the throat of the patient. The user pushes the knife assembly towards the throat of the patient. The cutting edge penetrates a tissue, or anterior wall of the throat, and enters the trachea, thereby providing an aperture, or stoma. The user transfers, or changes, the knife assembly into a second state, thereby providing the opening at the distal end of the knife and simultaneously dilating the aperture in the tissue. A tracheostomy device is placed inside the aperture via the opening. The tracheotomy apparatus is then removed from the aperture in the throat of the patient, leaving the tracheostomy device in place inside the aperture.

It is easier to manage, transport, store, the tracheotomy apparatus, and to overview the availability of the tracheotomy apparatuses in stock, compared to the conventional tracheotomy equipment. Moreover, only one single instrument, or unit, or system, or apparatus, is required for providing a tracheostomy, which makes it easier from logistics perspective. This means that the tracheotomy apparatus makes it easier to supply the healthcare facilities with the equipment needed for the tracheotomy procedure as such. It is understood that conventional surgical equipment such as sterile cloths, compresses, medicaments, anaesthesia equipment, sutures does not have to be included in the tracheotomy apparatus but may be provided separately. As the system may be packed as one unit in a packaging, only a single packaging per procedure has to be handled in the logistics chain, thereby making it easier to manage, transport, store and have an overview over the available equipment in stock. Moreover, having a single packaging reduces the environmental impact.

Alternatively, the tracheotomy apparatus may be packed in separate, or different, packaging. For example, the knife assembly may be packed in a packaging different to a knife support packaging. Packaging parts of the knife support system may result in a more compact packing of several packages in a transport or storage container. Thereby, a decreased environmental impact may be achieved.

The tracheotomy apparatus is advantageous compared to conventional tracheotomy equipment in that it makes it possible to provide a tracheotomy in a comparably short time. A possibility of providing a tracheostomy quickly is especially advantageous in an acute situation. In such situation it is essential that both the preparation and the procedure itself are performed as quickly as possible, taking security in consideration. Alternatively worded, the proposed tracheotomy apparatus reduces procedure duration as the user is provided with the equipment needed for the tracheotomy procedure in a single unit. The preparation time prior to the tracheotomy procedure is also reduced, as only one unit has to be handled by healthcare staff during preparation. For example, the time for retrieving the equipment needed from stock, opening the packaging, and putting it in order at a surgical table is decreased. Further, the system provides a possibility of uniformity of the shape and size of the tracheostomy, or stoma, independent on patient or surgeon (user). That is made possible by the predetermined dimensions and operation of the tracheotomy apparatus. Therefore, the dimensions of the tracheostomy are predictable and well-defined.

Further, it is easier to the choose other instruments required for tracheostomy, such as tubes, and for tracheostomy care, as the dimensions and operation of the tracheostomy are predictable and well-defined. That may further improve the logistics chain.

Moreover, the proposed fewer persons are required for the procedure, as all the steps of tracheotomy may be performed by a single user, in contrast to conventional procedures involving several parts/ instruments that require assisting staff.

The user is herein defined as a surgeon, a physician, or other person having an adequate education, performing the tracheotomy procedure.

The expressions "proximal" and "distal" are to be understood in relation to the user while the tracheotomy apparatus is in use. Alternatively worded, "proximal" means nearest to the user, and "distal" means farthest from the user, while the tracheotomy apparatus is in use. It is understood that the user may use the tracheotomy apparatus directly, or manually, or indirectly, such as by using robot assistance.

It is understood that the expression "assembly" has a common meaning of parts that are fitting together into a complete structure, or unit.

It is understood that in the second state a passageway is formed by the knife assembly.

It is understood that transverse extension or cross-sectional area at the distal portion of the knife increases when going from the first state to the second state. Alternatively worded, the transverse extension or cross-sectional area at the distal portion of the knife is greater in the second state than in the first state.

It is understood that in the second state the knife members are transversely separated, entirely or partially, in different directions.

The transverse extension or cross-sectional area may be fixed, or the same along the distal portion of the knife in the first state. The transverse extension or cross-sectional area may be smaller along the distal portion of the knife compared to the proximal portion of the knife. Additionally, or alternatively, the transverse extension or cross-sectional area may decrease from the proximal portion to the distal portion. In other words, the transverse extension or cross-sectional area may be smaller at the distal portion than at the proximal portion.

It is understood that the knife support is configured to support the knife both in the first state and the second state. "To support" means herein to hold up or serve as a foundation or prop. Thus, the knife support serves as a prop for the knife.

The knife support may form a rigid structure. The knife, or the proximal portion of the knife, may be releasably supported by the knife support. The knife, or the proximal portion of the knife, may be slidably supported by the knife support.

The knife support may provide stability to the knife. It may be configured to releasably fix, or maintain, the knife assembly in the first state and in the second state Additionally or alternatively, it may be configured to guide, or transfer, the knife assembly from the first state to the second state. This allows for a single user performing the tracheostomy.

The knife support may be manufactured of a material different to the knife. The knife support may have different surface characteristics relative to the knife. Thereby, it may have other mechanical characteristics relative to the knife. For example, the knife support may have a rough exterior gripping portion, providing a comfortable and secure grip. Such rough portion may prevent the knife system from rotating or gliding in the user's hand.

As mentioned above, the proximal portion may be releasably attached to the knife support in the second state. That means that the proximal portion, and in extension the complete knife, may be released from the knife support in this state.

The knife may be sufficiently stiff in order to penetrate the tissue. The knife may be produced, partly or entirely, in steel, such as stainless steel, or in high carbon steel titanium, or a ceramic material.

The distal portion may have, or form, a smooth surface in the first state, which contributes to reduced tissue damage in use.

As mentioned above, the knife has a proximal, or rear, portion and a distal, or forward, portion having a distal, or forward, end. It is understood that the distal portion of the knife is intended to at least in part be inserted into the trachea. The proximal portion may be longer than a distal portion. The distal portion may be longer than a proximal portion. The distal portion may be elongated. The proximal portion may be elongated. The portions may be manufactured in same material, or in different materials. The transverse extension or cross-sectional area of the portions may be the same. The transverse extension or cross-sectional area of the distal portion may be smaller relative to the transverse extension or cross-sectional area of the proximal portion.

The knife may comprise other portions than the proximal portion and the distal portion. There may be an additional portion connecting the distal portion to the proximal portion. The distal portion may be directly connected to the proximal portion. As the distal portion and the proximal portion are interconnected, the movement of one of the portions results in a corresponding movement of the other portion.

As mentioned above, the proximal portion is attached to the knife support. Thereby, as the distal and the proximal portions are interconnected, the knife as a whole is supported by the knife support.

The term "end" is understood as "the part of an area that lies at the boundary", or "a point that marks the extent of something". Here, the distal end is a terminal point or an area of the distal portion, or the extreme part of the distal portion lengthwise. The distal portion may have a uniform width, or transverse extension, in the first state.

The distal portion may form a cylinder in the first state. Similarly, the distal portion may outline a cylinder in the second state. This means that the distal portion has a same transverse extension or cross-sectional area along the complete length of the distal portion in each state.

The distal direction is understood as the direction away from a user when the tracheotomy apparatus is in use. The proximal direction is opposite to the distal direction and is understood as the direction toward the user when the tracheotomy apparatus is in use. It is further understood that the distal direction is relative the knife support in the description of the distal, or forward, end, and extending from the knife support.

As mentioned above, the knife, or the proximal portion of the knife, may be releasably supported by, or attached to, the knife support. This means that the knife support can be removed from the knife.

It is understood that the expression "cut" encompasses "to penetrate with or as if with an edged instrument". For example, a tissue may be divided into parts with an edged tool. Another example is a skin incision, where the wound is a cut made by incising the skin, especially in surgery.

It is understood that the knife has at least a cutting edge. The cutting edge is understood to encompass an edge that is pointy, such as edges commonly used in biopsy needles. As mentioned above, the distal portion may form a cylinder in the first state. The cylinder may have an oblique shape at the distal end, or the distal end may define a plane that is slanted, or angled, relative to the cylinder. The oblique shape or the slanted plane may form the cutting edge. The knife may have a serrated cutting edge.

It is specified above that the distal portion comprises, or forms, an opening at the distal end in the second state. It is understood that the expression "opening" encompasses an "aperture". The opening may be circular, triangular, or quadrangular. It may have a polygonal shape. The shape may be irregular.

The distal portion is configured to receive a tracheostomy device through the opening at the distal end in the second state. This means that the dimensions of opening are adapted in such way that tracheostomy device is allowed to be inserted in the opening.

The tracheostomy device may be a tube, a needle, a surgical instrument, or a catheter. The tube may be a tracheotomy tube, which is understood as a tube that is inserted into a tracheostomy, or stoma. The tube may be curved. The tube may have an inflatable cuff. The tube may be a fenestrated tube. For example, the tube may be a metal tube, or the tube may be made from polyvinyl chloride (PVC), silicone, or polyurethane.

The tracheostomy device may be a needle, such as a biopsy needle. The needle may be used for sampling or injection.

The tracheostomy device may be a surgical instrument, such as scalpel, scraper, or forceps. It may alternatively be a device that may impact tissue by for example heating, or laser.

The tracheostomy device may be a catheter. The catheter may be a catheter used for suction, or for applying liquids to the tissue.

The system may be configured to be sterilized, entirely or partially, for example, in autoclave. Therefore, the materials of the system, or parts of it, may be configured to resist high temperature and/or other sterilizing methods without being deformed, or in other way impacted such as to become inappropriate for usage. For example, the knife may be configured to be sterilized and reused, but the knife support may be disposable. Alternatively, the knife may be configured to be disposable, but the knife support may be sterilizable and reusable. Alternatively, the entire system may be disposable.

The tracheotomy apparatus may further comprise an opening mechanism. The opening mechanism may comprise an actuator operationally connected to the knife assembly. The opening mechanism may be configured to transfer, or change, the knife assembly from the first state to the second state.

The opening mechanism contributes to a controllable transfer between the first state and the second state.

The opening mechanism may comprise an actuator configured to transfer, or change, the knife assembly from the first state to the second state. The actuator may be manually operated or powered. The user thus has a better control while changing the knife assembly from the first state to the second state.

The actuator may comprise a rotating part configured to transfer, or change, the knife assembly from the first state to the second state at a rotational action of the rotating part relative to the knife assembly. The rotating part may be rotated or screwed around a rotational axis. The knife support may define a central axis extending in the proximal direction, or along the extension of the knife in distal direction. The rotational axis may be parallel, or coaxial, with the central axis of the knife support, or with the distal portion of knife.

Alternatively, or additionally, the actuator may be configured to transfer, or change, the knife assembly from the first state to the second state at a linear or pivotal action of the actuator, for example, the actuator may comprise a pair of arms that transfer, or change, the knife assembly from the first state to the second state when pushed together. In this alternative, the arms provide a security mechanism. Each arm may comprise a handle, or grip. The arms may be releasably locked, or biased, to maintain the knife assembly in the first state.

The knife may comprise, or be composed of, at least three elongated knife members. Each knife member forms part of the proximal portion and the distal portion of the knife. Here, the term "member" is understood as one of the individuals composing a group. Alternatively worded, the at least three knife members form together the knife, or the knife members may be separable.

In the first state the knife members may be juxtaposed, or in contact, in, or along, the distal portion. In the first state, the knife members may be spaced apart in, or along, the proximal portion of the knife. The knife may comprise an intermediate portion connecting the proximal portion and the distal portion of the knife.

Each knife member may comprise a first portion, a second portion, and a third portion. The first portions of the knife members jointly form the proximal portion of the knife, the second portions of the knife members jointly form the intermediate portion of the knife, and the third portions of the knife members jointly form the distal portion of the knife.

In the first state, the first portions may be parallel and spaced apart. The third portions may be parallel and juxtaposed to each other. The second portions may converge in the distal direction. Worded differently, the second portions may meet at the third portions.

In the second state, the first portions may be parallel and spaced apart. The third portions may be parallel and spaced apart. The second portions may converge in the distal direction.

The knife members may have the same dimensions, form, and material.

The opening mechanism may further comprise a separator. The separator may be operationally connected to the knife assembly. Additionally, the separator may be operationally connected to the actuator, or may operationally connect the actuator and the knife assembly. The separator may be configured to engage the knife to transfer the knife assembly from the first state to the second state.

It is understood that a separator is an element providing a separation of other elements by an action. The separator may separate the knife members during the transfer, or change, from the first state to the second state, leaving the knife members spatially separated from each other in the second state. Alternatively worded, the separator is configured to set the knife assembly in the second state.

The separator may slidably engage the knife, or the knife members. It may be configured to force, or push, the knife members apart at a movement of the separator in the distal direction. The separation may be transverse to the distal direction. The separator may be configured to move linearly along the distal direction.

The separator may comprise grooves, or guiding chamfers, configured to individually engage, or provide support to, the knife members. This configuration provides further stability to the knife assembly. Further, it offers additional control of the position of the knife members.

The separator may have a cone shaped, or convex, distal portion. facing in the distal direction. The distal portion may define a terminal point of the separator. Such a form is advantageous for providing a smooth transfer of the knife assembly from the first state to the second state.

The actuator may have a first position in the first state of the knife assembly and a second position in the second state. This means that the actuator determines the position of the separator. The actuator may be configured to move, or push, the separator from the first position to the second position. Thus, the actuator cooperates with the separator in transferring the knife assembly form the first state to the second state.

In the second state, the separator may at least in part be positioned within the distal part of the knife. This means that the separator determined the transverse extension of the distal part in the second state.

The first position of the separator is understood as the position being nearer to the user relative to the second position. This means that when the separator is set in the first position, it is positioned at a longer distance from the cutting edge relative to when it is set in a second position.

The separator, or the distal portion of the separator, may engage, or push, against the second portions of the knife members at a movement of actuator from the first position to the second position. This is particularly advantageous if the separator has a cone shaped distal portion and the second portions converge in the distal direction.

In a more general view, the knife may have a portion with a conical internal shape in the first state matching the cone shaped distal portion of the separator. The knife members may form a space in the intermediate portion of the knife in which the distal portion of the separator is positioned. separator. The distal portion of the separator may be positioned at the intermediate portion of the knife in the first state. Additionally, the separator may at least in part be positioned inside the proximal portion of the knife.

The separator may be configured to be inserted, or pushed, into the distal portion for forcing the knife to transfer from the first state to the second state.

As described above, the actuator may be manually operated, or powered. Additionally, or alternatively, the actuator may be mechanically operated, or powered, for example by a biasing component, such as a compression spring. For example, the spring may initially be in a contracted state. The actuator may be operationally connected to a spring holding arrangement which controls the expansion of the spring. The expansion is limited by the dimensions of the spring. The holding arrangement may have holding members that are slidably attached to the spring and glide along the spring upon activation of the actuator. Thereby, the spring may push the separator from the first position to the second position.

The knife assembly may further comprise an indicator. The indicator may be configured to present a signal to a user at contact between the cutting edge and the tissue during use. The signal may be an audible sound.

The indicator may comprise an elongated member that extends along the knife. The distal portion of the knife may form a through-going channel in first state that extends in the distal direction, and the elongated member may extend through the channel. The channel may be formed by the third portions of the knife members. The channel may have closed sides forming a tube-like structure. The elongated member may extend along the entire length of the knife members. The actuator may form an additional channel through which the elongated member extends.

The elongated member may be slidably supported by the knife.

The elongated member has a primary, or distal, position and a secondary, or proximal, position in relation to the knife. The elongated member may be biased to be in the primary position.

The elongated member may have a distal portion that, in the primary position, extends past the cutting edge at the distal portion of the knife in the distal direction. Additionally, in the secondary position, the cutting edge may extend past, or be abreast with, the distal portion in the distal direction. In the primary position, the distal portion may extend from the cutting edge by 1-15mm, 1-10mm,1-7mm, 1-5mm,1-3mm, or 1mm. The indicator may be configured to generate an audible first sound at a transition, or change, from the primary position to the secondary position. For example, this may be at contact with tissue during use. Similarly, the indicator may be configured to generate an audible second sound at a transition, or change, from the secondary position to the primary position. For example, this may be after passing through tissue during use.

The indicator may comprise an audible member operationally connected to the elongated member. The audible member is configured to produce an audible sound at a transition, or change, between the primary position and the secondary position of the elongated member. For example, the audible member may comprise, or be composed of, a plate, such as a metal plate, wherein the elongated member is operationally connected to the plate, for example by direct contact, and arranged to cause the plate to buckle and emit an audible sound in a transition, or change, from the primary position to the secondary position, and vice versa. The plate may further be arranged to bias the elongated member in the primary position.

The purpose of the indicator is to indicate a position of the cutting edge. Initially, when the user grips the knife support, the elongated member is in the primary, or distal, position. When the knife is pressed to a throat of a patient, the elongated member is pushed to the secondary, or proximal, position and an audible sound is produced by the audible member. Further, when the cutting edge has penetrated the tissue, and thereby passed the tissue and entered the air space of the trachea, the cutting edge is positioned inside the trachea. The elongated member is then pushed back into the primary, or distal, position and another audible sound is produced by the audible member. The tracheotomy apparatus is further pushed into the trachea. When the distal end of the elongated member reaches a tissue, for example the opposite side of the trachea, the distal end of the elongated member is pushed to the secondary, or distal, position, and an audible sound is again produced.

Thus, the user is made aware of the position of the cutting edge by counting the number of the audible sounds. Preferably, the sounds may differ from each other. Alternatively, there may be a first sound and a second sound, being different from each other. The first sound indicates transfer of the elongated member from the primary position to the secondary position. The second sound indicates transfer of the elongated member from the secondary position to the primary position.

The knife support may be configured to guide the knife members transversely outwards, preferably radially outwards, in a transition, or change, from the first state to the second state.

Worded differently, the knife support may be configured to limit the movement of the knife members to a radial expansion in a transition from the first state to the second state.

Such a guidance is advantageous as it provides further control of the movement of the knife members when the knife assembly changes state. The knife support may comprise a support base. The support base may form apertures, or channels, preferably radially extending apertures, or channels. Each aperture may be configured to guide a single knife member of the at least three knife members at a transition, or change, from the first state to the second state of the knife assembly.

The knife members may be releasably and/or slidably supported by the support base.

Each aperture may be formed to guide the single knife member radially outwards.

Each aperture may have a first edge to which the proximal portion of the knife is connected when the knife assembly is in first state. Each aperture may have a second edge radially spaced apart from the first edge and to which the proximal portion of the knife is connected when the knife assembly is in the second state. The aperture may comprise least two lateral edges that together with the first and second edges forms the aperture.

Each knife member may extend through respective aperture and engages the support base at the aperture.

The apertures may have different dimensions. Alternatively, some of the apertures may share dimensions, and other may have different dimensions. Preferably, two of the apertures are longer, or have longer lateral edges relative to the remaining aperture, or apertures. The longer apertures are neighbouring apertures, which means that there is no other aperture positioned between them.

The apertures may be elongated. The apertures may differ in form. The apertures may be straight. The apertures may have a locking, or securing, portion. The securing portion has an obstacle, preferably at a lateral edge, that the knife member has to overcome, or pass by, for example by an additional movement of the knife member. The additional movement may differ in direction relative to the remaining movement of the knife member at transfer from the first state to the second state.

The apertures may be interconnected. For example, the support base may form a central aperture interconnecting apertures.

The apertures may be interconnected on at least one lateral edge.

Each aperture may comprise a holding section, or inner section, and a release section, or outer section, radially distant from the holding section. Each knife member is positioned at the holding section in the first state of the knife assembly. Each of the apertures may have constant width between the lateral edges at the holding section. Alternatively worded, for each aperture, the aperture may have a same width at the portion of the aperture, wherein a knife member is positioned when the knife assembly is in the first state and between the first state and the second state.

Each knife member is positioned at the release section in the second state of the knife assembly. The release section of each of the aperture may have a larger width, or distance between the lateral edges of the aperture, compared the holding section. Alternatively worded, each aperture may comprise, or have, a wider portion, or releasing portion, which is configured to allow passage of respective knife member in the distal or proximal direction.

It is understood that the support base may be released from the knife support in the second state of the knife assembly. The support base may by connected to the actuator. The separator may be attached to the support base. The support base may be pushed in the distal direction, away from the knife support, by the actuator.

Each knife member may extend through respective aperture of the support base, and comprise, or form, a fastening protrusion, preventing the knife member from being released from the support base in the first state and in transition from the first state to the second state. Alternatively worded, for each knife member, the first portion may extend through one of the apertures. This is understood to be in the proximal direction relative to the cutting edge. The first portion of the knife member may form a fastening protrusion. The fastening protrusion may extend tangentially relative to the positions of the holding sections and the release sections, or relative to the central axis.

It is understood that when released the respective knife member is no longer extending through respective aperture of the support base. In other words, when released, the knife forms a unit being separate from the knife support.

The fastening protrusion and the holding section of the aperture may cooperate to prevent the knife member from being removed in the distal direction in the first state of the knife assembly. The release section may allow passage of the fastening protrusion in the distal direction in the second state of the knife assembly, thus allowing for the knife to be removed from the knife support.

The length of each of the fastening protrusions, or fastening portions, may be greater than the width of the corresponding apertures in the first state and between the first state and the second state. Alternatively worded, the length of each of the fastening portions may be greater than the width of the holding section of corresponding aperture. Each aperture, which may be radially or linearly extending, may comprise, or have, a wider portion configured to allow passage of the respective, or corresponding, fastening protrusion.

It is understood that the fastening protrusions, or fastening portions, may form hooks, or angle brackets, or L-squares, that are slidably connected to the knife support. The fastening protrusions may have same dimensions. The fastening protrusions may extend tangentially. The fastening protrusions may be directed, or pointing, in any direction that provides a support from the knife support when the knife is not in the second state. The fastening protrusion may be composed of the same material as the rest of the knife member.

The knife support may further comprise a locking member, or a locking disc. The locking member has a blocking state in which it, for each knife member, blocks, or prevents passage of the fastening protrusion through the respective aperture of the support base when the knife assembly is in the first and the second state. Alternatively worded, for each knife member, in the blocking state the fastening protrusion and the locking member cooperate to prevent the knife member from being released from the knife support in the second state of the knife assembly.

The locking member may further have a releasing state in which, for each knife member, the locking member allows passage, or releases, of the fastening protrusion through the respective aperture of the support base when the knife assembly is in the second state. Alternatively worded, for each knife member, in the releasing state the locking member allows passage of the fastening protrusion through the release section.

The locking member may be slidably supported relative to the support base. The locking member may be configured to be positioned juxtaposed to the support base.

The locking member may be configured to rotate around the central axis in relation to the support base. Transferring the locking member from the blocking state to the releasing state may be performed by a rotational movement of the locking member, preferably around the central axis. Worded alternatively, the locking member may change from the blocking state to the releasing state by a movement, preferably, a rotational movement, relatively to a support base. The rotation may be in a range of 1°-180° around the central axis, 1°-120°, 1°-90°, 1°- 80°, 1°- 60°, 1°-45, 1°-30°, 1°-20°, 1°-15°, 1°-10, more preferably 10°-120°, 10°-90°, 10°- 80°, 10°- 60°, 10°-45, 10°-30°, 10°-20°, 10°-15°, most preferably 1°-30°. The linear movement may be performed over a span of 1-10 mm. The locking member may comprise a manual interface. It is understood that the rotational movement may be performed by manual manipulation of the manual interface of the locking member.

The locking member may comprise apertures. The apertures may have a form of a circle sector, or a segment of a circle sector. The apertures may have a form of a square or be irregular. The apertures of the locking member may be configured to be positioned over, or overlay, the corresponding apertures of the support base in. Thereby, the fastening protrusion of each knife member is slidably connected to the locking member when the locking member is in a blocking state, and the knife assembly is not released from the knife support. Alternatively worded, the locking member may be positioned between the fastening protrusions of the knife members and the support base. Thereby, the locking member prevents the passage of the fastening protrusion of the knife member through the release section of the aperture of the support base when the locking member is in the blocking state.

It is understood that if the knife members are interconnected, at least one fastening protrusion is supported by the locking member. Alternatively worded, if the knife members are interconnected, the support is provided to at least one of the fastening protrusions. Thereby, the remaining knife members are supported indirectly by the locking member in the blocking state.

For at least one of the apertures of the locking member, the locking member may have a varying thickness along its edges, or form a slope. For example, the thickness may increase by 1mm, 2mm, 5mm, or 7mm, when going from the holding section to the release section. This is beneficial as it allows positioning the knife members not in line as at the second state, as seen in the transversal direction. Worded alternatively, the fastening protrusions of the at least one knife member may be lifted, or being in an elevated position, in the proximal direction relative to other knife members at the second state.

The tracheotomy apparatus may further comprise a base structure. The base structure may be located at the distal portion of the knife, or at the distal end of the distal portion of the knife.

The base structure may be arranged to stabilise the tracheotomy apparatus. It may further be arranged to support to the knife. During use, the base structure is intended to be in contact with the throat of the patient, thereby providing further support to the user.

The base structure may comprise a base aperture. The distal portion of the knife extends through the base aperture of the base structure. The base aperture may have an open side configured to allow the base structure to be removed from the knife in a transverse direction in the second state of the knife assembly.

In the first state of the knife assembly, the knife is movable relative to the base structure in the distal direction. Additionally, the base aperture may be arranged to provide radial support to the knife members in the second state of the knife assembly.

The base structure may comprise an additional knife support at the base aperture. The additional knife support may extend in the proximal direction.

The separator may comprise a releasable clip configured to bias the knife members against the additional knife support in the second state of the knife assembly. The releasable clip may be configured to be released and lock to the knife support at the second position of the separator. This way, the knife members are supported and maintained in the position of the second state after removal of the knife from the knife support of the knife assembly.

The tracheotomy apparatus may comprise a targeting plate attached to the base structure and extending in the distal direction.

The targeting plate is intended to be used for guiding the direction or position of the assembly in use. The guiding plate provides a possibility of positioning the tracheotomy apparatus at the throat of the patient, or alternating the position of the tracheotomy apparatus, prior to penetrating the tissue, and thereby without damaging the tissue if the position shows to be less desirable.

The targeting plate may have a distal edge. It is understood that the distal edge of the targeting plate is intended to be placed at the throat of the patient. The distal edge of the targeting plate may be formed to fit between the tracheal rings.

The distal edge of the targeting plate may extend beyond the cutting edge prior to the cutting edge penetrating the tissue. Alternatively worded, the extension of the targeting plate from the base structure may be longer than the extension of the knife members from the base structure, or equally long. The targeting plate may preferably extend parallelly to the distal portion of the knife.

The distal edge of the targeting plate may be concave. This way the distal edge conforms to the shape of the throat.

The targeting plate may be manufactured from the same material as the base structure.

The targeting plate may be releasably attached to the base structure, by means for fastening, for example by a click-fit or by mechanical friction.

The base structure may be configured to guide the targeting plate in the proximal direction subsequent to the targeting plate being released from the base structure. At the first state of the knife assembly, the additional knife support may slidably support the targeting plate subsequent to the targeting plate being released. Targeting plate may be released from the base structure at the second state of the knife assembly.

The base structure may comprise, or be releasably attached to, a spring mechanism configured to releasably fix the base structure to the knife support.

The spring mechanism may comprise a blade spring. The blade spring may have a central rounded, or curved, portion and connecting radially extending portions. The radially extending portions may be accessible from outside the tracheotomy apparatus.

The spring mechanism may be in a loaded position and biased to be in a released position. The spring mechanism may be configured to be manually released from the loaded position, for example by the user engaging the radially extending portions of the blade spring.

The spring mechanism may be operationally connected to the to the locking member of the knife support. The spring mechanism may be configured to transition, or change, the locking member from the blocking state to the releasing state at a change from the loaded position to the released position.

For example, the spring mechanism and the locking member may be connected by a connecting member operationally connecting the locking member of the knife support and the spring mechanism. Preferably, the connecting member is elongated. It may be rigidly attached to the blade spring. This means, that a movement of the blade spring impacts the state of the locking member.

The spring mechanism may further comprise a steel blade being in mechanical connection to a blade spring. Preferably, the steel blade is positioned radially outwardly relative to the blade spring. The steel blade positions the blade spring and tensions it in direction toward the steel blade.

The base structure may form a casing support extending in the proximal direction. The casing support may be formed to conform with at least a part of the blade spring, preferably at least when the spring mechanism is not actuated. Alternatively worded, an internal surface of the blade spring may have, at least partially, a form of an exterior surface of the casing support of the base structure. The casing support may comprise high friction surface. The casing support may be formed as an elongated proximally-extending protrusion. Preferably, casing support is spaced apart from the additional knife support, more preferably in radial direction. The casing support may be curved, such as a circle sector, as seen in a transverse cross-section. Alternatively, the casing support may be rectangular. The casing support may have grooves on at least one side.

The tracheotomy apparatus may further comprise an exterior casing releasably supporting the base structure relative to the knife support. The exterior casing may engage the casing support of the base structure. It is understood that the expression "exterior" means situated outside, or having merely the outward appearance. The term "casing" means having merely the outward appearance, such as an enclosing frame. The casing may comprise two or more separate parts, being movably, or slidably attached to each other.

The exterior casing may be positioned in the proximal direction relative to the base structure. The casing may be releasably attached to the base structure.

The casing is advantageous as it provides further support to the user. The casing may be configured to have a rough exterior surface providing a good hold during use tracheotomy apparatus.

Preferably, the casing may have a cylindrical form. In case the casing comprises two or more separate parts, it is preferable that at least one of the parts has a cylindrical form. Additionally or alternatively, one of the parts may be formed as a sector of a circle.

The casing may have a proximal aperture through which the actuator may extend. The casing may have lateral apertures through which the blade spring extends.

It is understood that the casing has a distal opening at the base structure through allowing passage of the knife.

The casing may comprise a bottom part. The bottom part may be releasably attached to the base structure by friction. Alternatively or additionally, the bottom part may be attached to the base structure via grooves and flanges. The bottom part and the base structure may in part overlap

The blade spring, preferably in combination with the steel blade, may be arranged bias the base structure and release the bottom part from the base structure by manual action of the blade spring. Alternatively, if the bottom part may be attached to the base structure via grooves and/or flanges, the release may be performed by separating the flanges from the grooves.

The bottom part may be arranged to prevent removal of the targeting plate. When the bottom part is released from the base structure, the targeting plate may also by removed.

The knife assembly may further comprise a flexible, or deformable, mantle, or expandable tube, flush with and extending along at least a portion of the distal portion of the knife. The mantle is configured expand radially at the transition from the first state to the second state of the knife assembly. The term "mantle" is understood as an element that covers or envelops.

The mantle may be attached to the knife members. The knife members may jointly form an exterior surface of the distal portion of the knife in the first state of the knife assembly. Alternatively worded, the third portions of the knife members may jointly form an exterior surface. The mantle may be attached to, or bias itself against, the exterior surface of each of the knife members.

The mantle may be configured to support the knife members and prevent them from deviating, or separating, in the first state of the knife assembly. This function may also be present in the second state. The mantle has an outer surface that presses against the tissue after the knife has been inserted. This contributes to a reduced bleeding, in particular when the knife assembly is in the second state. The mantle may be pre-processed, such as covered, with a coagulant, such as metallic salt or cationic polymer.

Preferably, the mantle is manufactured from silicone, latex, polyvinyl chloride (PVC), or polyurethane.

The mantle may initially be formed as a cylinder. The mantle may be configured to rupture, or burst, at transfer from the first state to the second state of the knife assembly. The mantle may be configured to burst, or rupture, between two of the knife members at transfer from the first to the second state. The mantle may comprise a perforation extending in the distal direction between a pair of neighbouring knife members. The mantle may be configured to rupture along the perforation. Preferably, the perforation is formed as serial triangles. The rupture provides for an easier positioning of a tracheostomy device.

### Brief description of the drawings

A more complete understanding of the abovementioned and other features and advantages of the proposed technology will be apparent from the following detailed description of preferred embodiments in conjunction with the appended drawings, wherein:
Figures 1A and 1B illustrate a perspective view of an embodiment of a tracheotomy apparatus when a knife assembly is in a first state.
Figure 2A schematically illustrate an embodiment of a knife when a knife assembly is in a first state.
Figures 2B and 2C schematically illustrate an alternative embodiment of a knife when a knife assembly is in a second state.
Figure 3A and 3B schematically illustrate an embodiment of a tracheotomy apparatus when a knife assembly is in a first respectively a second state.
Figure 3C schematically illustrates an embodiment of a knife and a base structure when a knife assembly is in a second state.
Figure 4A,B and 5A,B schematically illustrate an embodiment of a tracheotomy apparatus when a knife assembly is in a first respectively a second state.
Figure 4C is a top view of an alternative embodiment of a tracheotomy apparatus when a knife assembly is in a first state.
Figure 6A schematically illustrates an embodiment of an indicator.
Figures 6B and 6C schematically illustrate an embodiment of a tracheotomy apparatus having an indicator.
Figures 7A-7C schematically illustrate an embodiment of base structure, a target plate respectively a combination thereof.
Figures 7D-7F schematically illustrate an embodiment of a tracheotomy apparatus with a spring mechanism.
Figure 7G is an exploded view of an embodiment of a tracheotomy apparatus having an exterior casing.
Figures 8A,B,C-11A,B,C together schematically illustrate how a knife assembly of a tracheotomy apparatus is transitioned between a first state and a second state.

### Description of the drawings

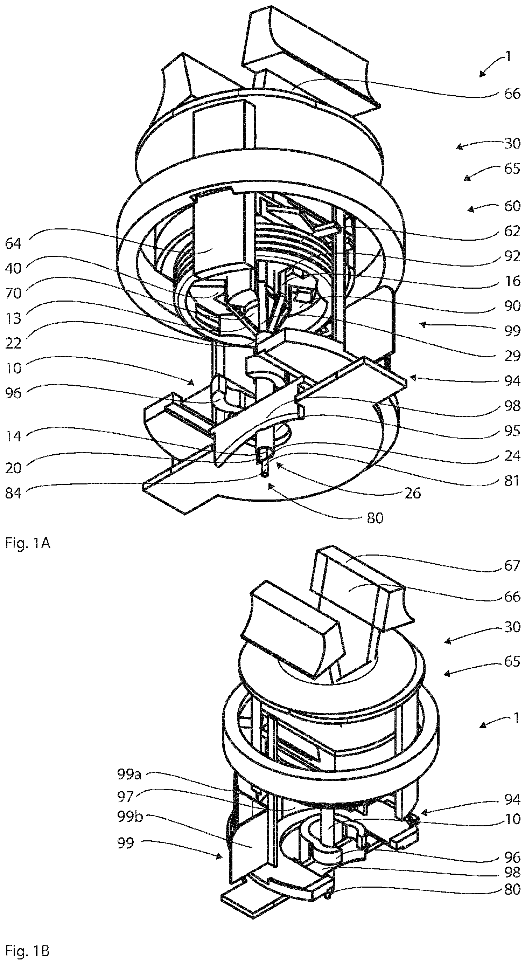

Figures 1A and 1B schematically illustrate an embodiment of a tracheotomy apparatus 1 having a knife assembly 30 in a first state, which means that a knife 10 is not expanded. The knife assembly 30 is in the first state prior to the tracheotomy procedure, and prior to dilating the opening in the tissue when the knife 10 is inserted in the tissue.

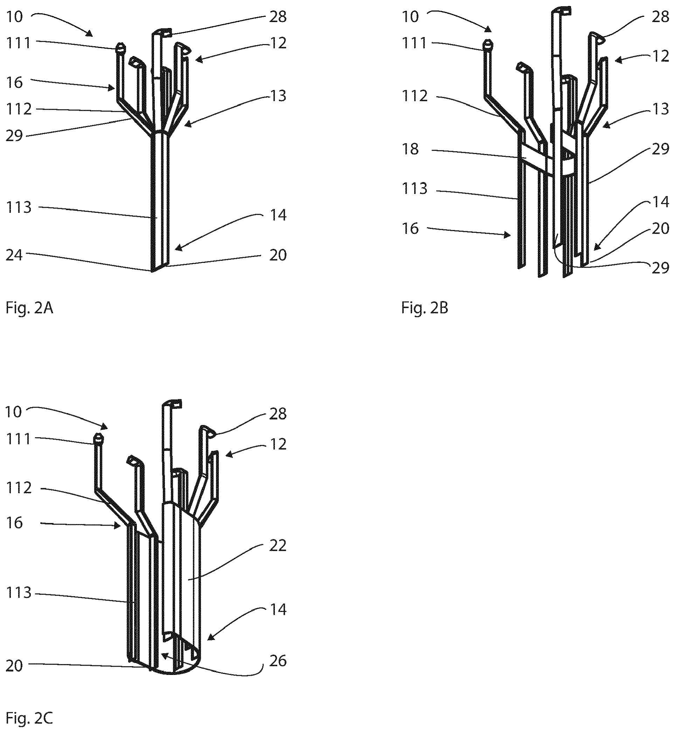

The knife 10 is made of steel, is sufficiently stiff and has a proximal portion 12 (shown in Figs. 2A-C). The knife 10 also has a longer distal portion 14 having a smooth surface in the first state. The proximal 12 and the distal 14 portions are manufactured in the same material. The distal portion 14 has a uniform width and forms a cylinder in the first state.
The transverse extension or cross-sectional area of the distal portion 14 is smaller relative to the transverse extension or cross-sectional area of the proximal portion 12. Further, the knife 10 has an intermediate portion 13, which interconnects the proximal portion 12 and the distal portion 14. The distal portion 14 is elongated and has a distal end 20. The cylinder formed by the distal portion 40 has an oblique shape at the distal end 20. The distal portion 14 of the knife 10 forms a cutting edge 24 at the distal end 20. The cutting edge 24 is intended to be the edge that penetrates the tissue. The knife 10 has elongated knife members 16, all manufactured of the same material. In first stat, the knife members 16 are juxtaposed along the distal portion 14 and together form the knife 10. Each knife member has a first portion 111, a second portion 112, and a third portion 113, as illustrated in Figures 2A-C. In the first state, the first portions 111 are parallel and spaced apart. The third portions 113 are parallel and juxtaposed to each other. The second portions 112 converge in the distal direction. In a second state, the first portions 111 and the third portions 113 are parallel and spaced apart.

Figures 1A-1B further show a mantle 22, interconnecting the knife members 16, thereby preventing them from separating in the first state of the knife assembly 30. The mantle 22 is attached to an exterior surface 29 (shown in Figs. 2A-B) of each of the knife members 16. The mantle 22 is manufactured from silicone and is covered with a coagulant.

The mantle 22 forms a cylinder when the knife assembly 30 is in the first state. The mantle 22 is configured to burst, or rupture, between two of the knife members 16 at transfer from the first to the second state (compare Figs. 1A and 2C) along a perforation (not shown) extending in the distal direction between a pair of neighbouring knife members 16. As shown in Figure 2C, the mantle is ruptured between two of the knife members 16 along the perforation when the knife assembly is in the second state.

Figures 1A-1B further schematically illustrate that the proximal portion 12 of the knife 10 is attached to a knife support 40. Optionally, the knife support 40 has a rough exterior gripping portion (not shown). The knife support 40 has a rigid structure. The knife 10 is supported by the knife support 40 in a releasable and slidable manner. The knife support 40 is manufactured of a material different to the material of the knife 10. The knife 10 and the knife support 40 form together the knife assembly 30.

The intermediate portion 13 of the knife 10 has a conical internal shape and encompasses a separator 70 having a cone shaped distal portion. The second portions 112 of the knife members 16 are formed to fit the exterior of the separator 70. The separator 70 has grooves (not shown), which are optional.

Subsequently to the knife 10 has penetrated the tissue and been positioned in the desired position, the knife 10 is dilated. The dilation is performed by moving the separator 70 in distal direction. In the embodiment illustrated in Figure 1A, the distally directed movement of the separator 70 is performed by a spring 62 of an actuator 60. In this embodiment, the actuator 60 has a rotating part 65 configured to transfer the knife assembly 30 from the first state to the second state at a rotational action of the rotating part 65 relative to the knife assembly 30. The rotating part 65 is rotated around a rotational axis (not shown). The spring 62 is initially in a contracted state, as illustrated in Fig. 1A. The spring 62 is connected to a spring holding arrangement 64 which controls the expansion of the spring 62. The spring holding arrangement 64 has holding members that are slidably attached to the spring 62 and glide along the spring 62 at a rotational movement of the actuator 60. Thus, the more the actuator 60 is rotated, the greater movement of the separator 70 in the distal direction is performed. The expansion of the spring 62, and thereby the distal movement of the separator, is limited by the dimensions of the spring 62. Thus, the separator 70 is pushed in the distal direction, dilating the knife 10.

The actuator 60 has a pair of arms 66 that enable transfer of the knife assembly 30 from the first state to the second state when pushed together. Each arm has a handle 67. In this embodiment, the arms 66 provide a security mechanism.

Figures 1A-1B further illustrate a support base 90 of the knife support 40. As described above, the knife 10 is attached to the knife support 40. The proximal portion 12 of the knife 10 extends through apertures 92 of the knife support 40. Figure 1A shows apertures 92 of the knife support 40, configured to guide knife members 16 outwards.

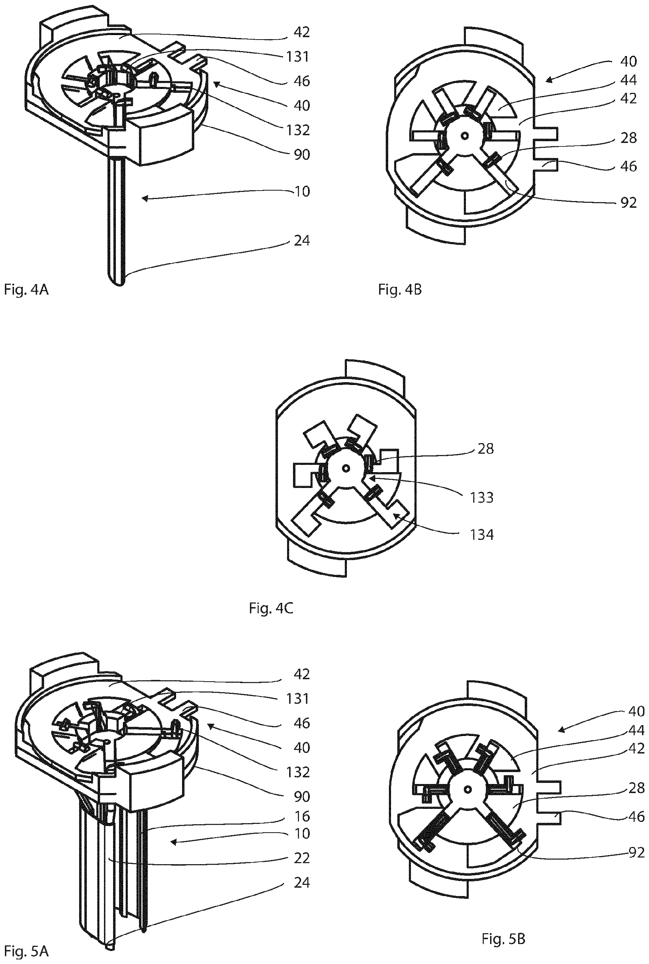

Figures 4A,B and 5A,B illustrate the apertures 92 in detail. Each aperture 92 has a first edge 131 and a second edge 132 radially spaced apart from the first edge 131. The aperture 92 has two lateral edges that together with the first 131 and second 132 edges form the aperture 92. The proximal portion 12 of the knife 10 is connected to the first edge 131 when the knife assembly 30 is in the first state. The proximal portion 12 of the knife 10 is connected to the second edge 132 when the knife assembly is in the second state. As illustrated in Figure 4C, each aperture 92 has a holding section 133, distant from a release section 134. Each knife member 16 is positioned at the holding section 133 in the first state of the knife assembly 30. The release section 134 of each of the aperture 92 has a larger distance between the lateral edges of the aperture 92, compared the holding section 133. The releasing portions 134 are configured to allow passage of respective knife member 16.

In an alternative embodiment, the apertures 92 have a locking portion (not shown).

The knife members 16 have each a fastening protrusion 28 (Figs. 2A-C), which prevents the respective knife member 16 from being unintentionally demounted from the knife support 40. A fastening protrusion 28 is composed of the same material as the rest of the knife member 16. The fastening protrusions 28 are supported by a locking member 42, illustrated in Figure 4B. Figure 4B further illustrates that the locking member 42 has apertures 44 through which the proximal portion 12 of the knife 10 extends. The apertures 44 of the locking member 42 are positioned over the corresponding apertures 92 of the support base 90. Optionally, the locking member 42 has a manual interface 46. The manual interface 46 of the locking member 42 is configured to be manually manipulated to transfer the locking member 42 from a blocking state to a releasing state. Figure 4C illustrates an alternative embodiment without the corresponding manual interface 46. In an alternative embodiment, the locking member 42 has a varying thickness, or form a slope (not shown).

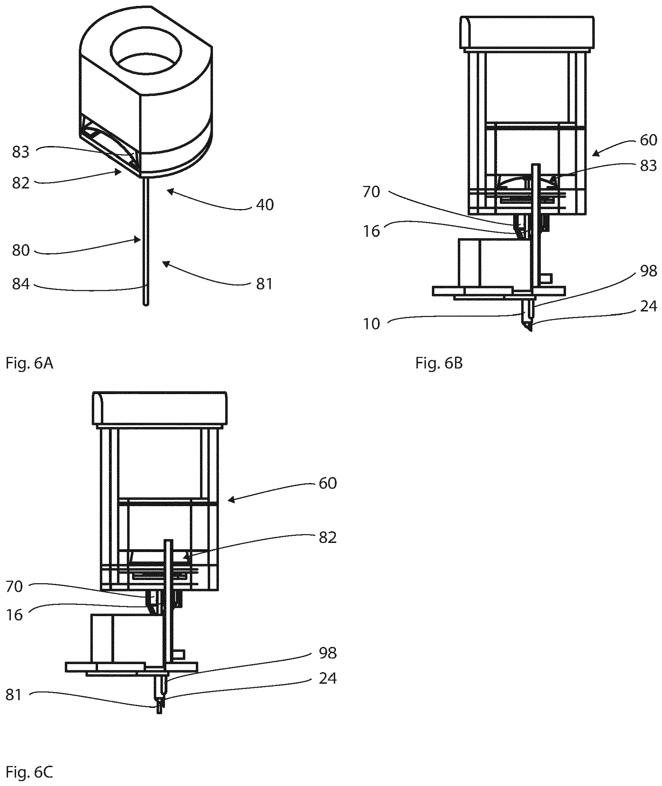

Figures 1A-1B further illustrate an indicator 80 in the primary position. An embodiment of the indicator 80 is illustrated in detail in Figures 6A-C. The indicator 80 has an elongated member 81 that extends along the knife 10. The distal portion 14 of the knife 10 forms a through-going channel in the first state that extends in the distal direction, and the elongated member 81 extends through the channel. The channel is formed by the third portions 113 of the knife members 16. The channel has closed sides forming a tube-like structure. The elongated member 81 extends along the entire length of the knife members 16. The actuator 60 forms an additional channel through which the elongated member 81 extends (not shown).

The elongated member 81 in a primary position is illustrated in Figures 1A, 6C. The elongated member in a secondary position in relation to the knife is illustrated in Fig. 6B. The elongated member has a distal portion 84 that, in the primary position, extends past the cutting edge 24 in the distal direction. In the secondary position, the cutting edge 24 extends past the distal portion 84 in the distal direction. The indicator 80 is configured to generate an audible first sound at a transition between the primary position and the secondary position. In the embodiment illustrated in Figures 6A-C, the indicator 80 is biased to the knife support 40 and is movable relative to the knife 10. The elongated member 81 extends along the knife 10. The elongated member 81 is partly encompassed in the through-going channel formed by knife 10.

The indicator has an audible member 82 connected to the elongated member 81. The audible member is configured to produce an audible sound at a transition between the primary position and the secondary position of the elongated member 81. In the embodiment illustrated in Figures 6A-C, the audible member 82 has a plate 83. The elongated member 81 is connected to the plate 83 and arranged to cause the plate 83 to buckle and emit an audible sound in a transition from the primary position to the secondary position, and vice versa. The plate 83 is further arranged to bias the elongated member 81 in the primary position.

Figures 1A-1B further schematically illustrate a base structure 94. The base structure has a base aperture 95. The distal portion 14 of the knife 10 extends through the base aperture 95. The base aperture 95 has an open side which allows the base structure 94 to be removed from the knife 10 in a transverse direction in the second state of the knife assembly 30. Figures 1A-1B further illustrate an additional knife support 96 which is positioned along a sector of the circumference of the base aperture 95.

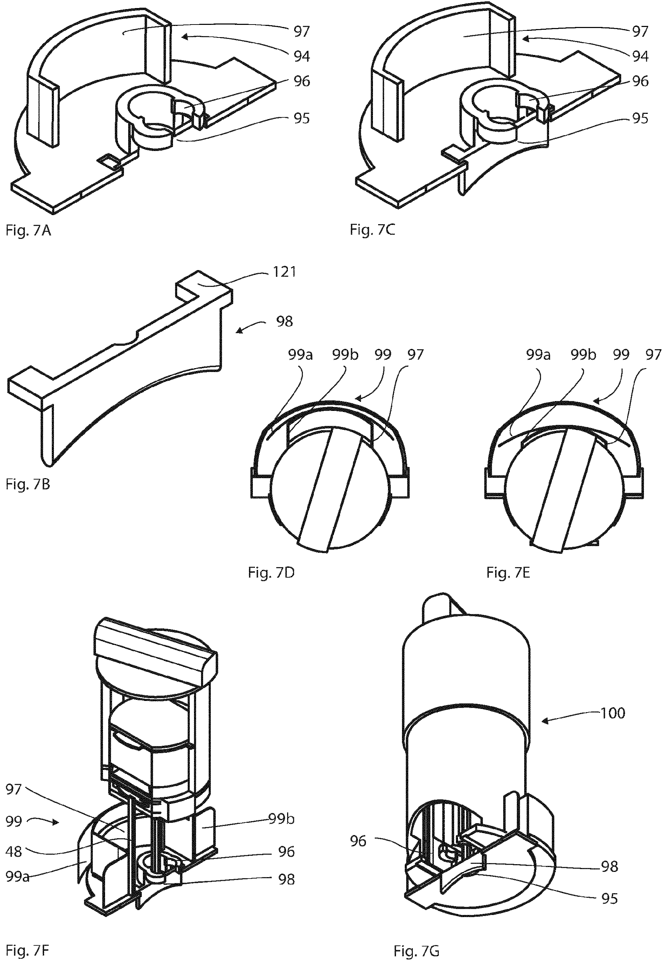

Figures 1A-1B further schematically illustrate a targeting plate 98 manufactured of the same material as the base structure 94. The distal edge of the targeting plate 98 is curved, to better fit to the form of the throat. The targeting plate 98 has means for fastening 121 (Figure 7B) to the base structure 94.

Figures 1A and 1B further schematically illustrate a spring mechanism 99. The base structure 94 is attached to the spring mechanism 99. The spring mechanism 99 has a steel blade 99a a and a blade spring 99b. The blade spring 99b has a form of omega (Ω), having a central rounded portion and radially extending edges. The radially extending portions are accessible from outside the tracheotomy apparatus 1.

The blade spring 99b is in mechanical connection to the steel blade 99a. The steel blade 99a is positioned radially outwardly relative to the blade spring 99b.

Figure 1B further illustrates a casing support 97. The casing support 97 of the base structure 94 is formed to conform with a part of the blade spring 99b. Thus, a part of the internal surface of the blade spring 99b has a form of an exterior surface of the casing support 97 of the base structure 94. The casing support 97 has a high friction surface (not shown).

Figures 2A-C schematically illustrate the knife 10 separate from the knife support 40. Figure 2A shows an embodiment of the knife 10 while the knife assembly 30 is in the first state. The knife 10 has six knife members 16. Figures 2B-2C show the knife 10 when the knife assembly 30 is in the second state. The first portions 111 of the knife members 16 jointly form the proximal portion 12 of the knife 10, the second portions 112 of the knife members jointly form the intermediate portion 13 of the knife, and the third portions 113 of the knife members jointly form the distal portion 14 of the knife 10.

Figure 2B further illustrates a releasable clip 18. As described above, the separator 70 (not shown in Figure 2B) has the releasable clip 18, which is configured to bias the knife members 16 against the additional knife support 96 in the second state.

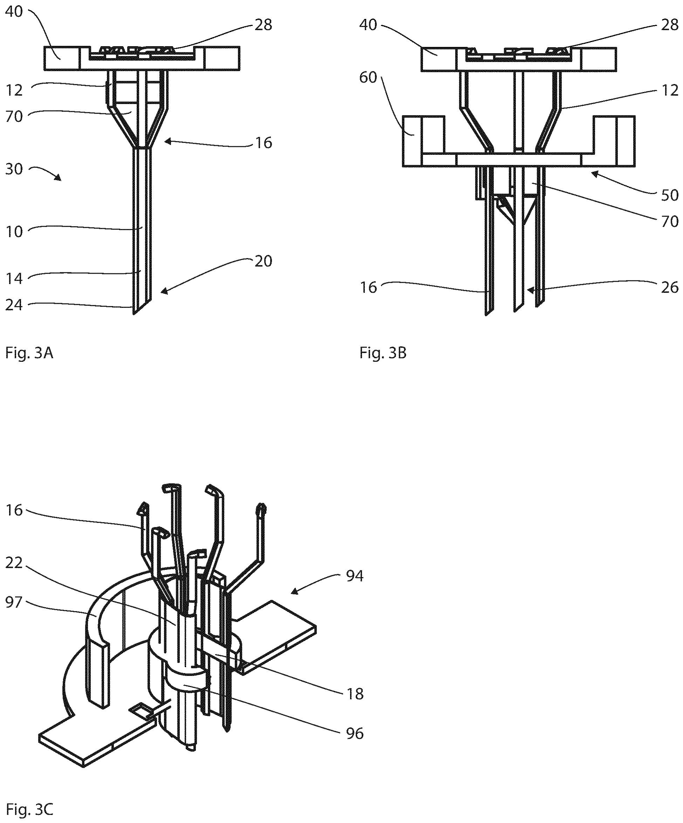

Figures 3A-B schematically illustrate an embodiment of a tracheotomy apparatus 1 having a knife assembly 30 in the first state respectively in the second state. Knife members 16 are attached to the knife support 40. In Figure 3B, an opening mechanism 50 has forced the knife assembly 30 in the second state by moving a separator 70 in the distal direction. The knife members 16 are, thus, separated, compared to the first state (Figure 3A). The distal portion 14 outlines a cylinder in the second state. A circular opening 26 at the distal end 20 is thereby provided. In this embodiment, the state transfer is enabled by screwing or pushing the actuator 60. Figure 3C illustrates that the releasable clip 18 biases the knife members 16 against the additional knife support 96, as illustrated in Fig. 3C.

Figures 4 A-B and 5 A-B provide a further illustration of the embodiment wherein the knife assembly 30 is in the first respectively the second state. Figures 4B and 5B illustrate a knife support 40 of the knife assembly 30, as seen in the distal direction. As illustrated in the Figures 4 A-B and 5A-B, the fastening protrusions 28 of knife members 16 are outwardly moved in the second state compared to the first state. Figures 5 A-B and 6A-B further illustrate apertures 92 of the knife support 40, and apertures 44 of the locking member, through which the proximal portion 12 of the knife 10 extends.

Figures 4A-B and 5A-B is a further illustration of the knife assembly 30 in the different states. Figure 4A schematically illustrates an embodiment of the knife assembly in the first state. Figure 5A schematically illustrates an embodiment of the knife assembly in the second state. Figure 5A differs from Figure 4A in that the knife 10 is expanded, or the knife member 16 are separated. Figures 4B and 5B illustrate the knife support 40 and the fastening protrusions 28, as seen in the distal direction. The fastening protrusions 28 have a more outward position at the knife support 40 in Figure 5B compared to Figure 4B. The fastening protrusions 28 are supported by the locking member 42. The mantle 22 is attached to knife members 16.

Figure 7A is a detailed illustration of an embodiment of the base structure 94. The base structure has the base aperture 95. The distal portion 14 of the knife 10 is intended to extend through the base aperture 95. Figure 7A further illustrates an additional knife support 96 which is positioned along a sector of the circumference of the base aperture 95. The additional knife support 96 supports the knife members as described above and shown in Figure 3C. Also, Figure 7A illustrates optional openings by means of which the targeting plate 98 is intended to fit the base structure 94. Means for fastening 121 are, in this embodiment, intended to be attached at the optional openings. Figure 7A further illustrates a casing support 97.

Figure 7B schematically illustrates the targeting plate 98. The distal edge of the targeting has a curved form, to better fit to the form of the throat. The targeting plate 98 has means for fastening 121 to the base structure 94. The means are here represented by protrusions.

Figure 7C schematically illustrates the targeting plate 98 mounted on the base structure 94. Figures 7A and 7C further illustrate the casing support 97. Figures 7D-E are top views of an embodiment of the tracheotomy apparatus 1. Figures 7D-F schematically illustrate an embodiment of the knife assembly 30. The spring mechanism 99 is shown in the Figures 7D-F. The base structure 94 is in contact with the spring mechanism 99. The spring mechanism 99 has a steel blade 99a a and a blade spring 99b.

In an alternative embodiment, as illustrated in Figure 7F, the spring mechanism 99 and the locking member 42 are connected by a connecting member 48. The connecting member 48 is elongated and is rigidly attached to the blade spring 99b.

Figure 7G schematically illustrates an exploded view of an embodiment of the tracheotomy apparatus 1 having an cylinder-formed exterior casing 100. The casing 100 may has a rough exterior surface providing a good hold during use tracheotomy apparatus 1. Here, the exterior casing 100 has two separate parts: a proximal part and a distal bottom part. The parts are attached to each other. The bottom part is attached to the base structure 94 via grooves (not shown). The exterior casing 100 has a proximal aperture through which the actuator 60 extends.

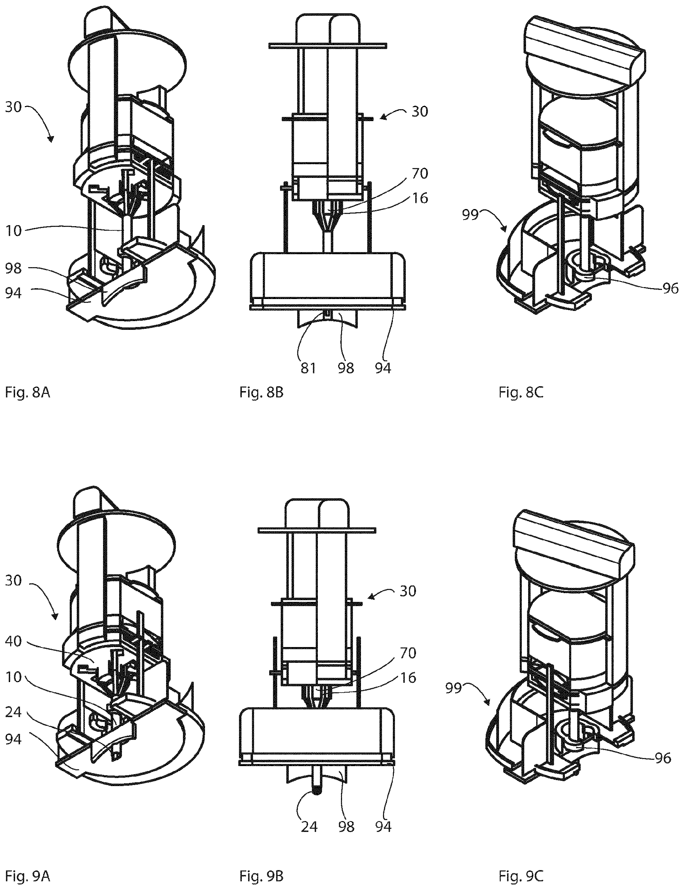

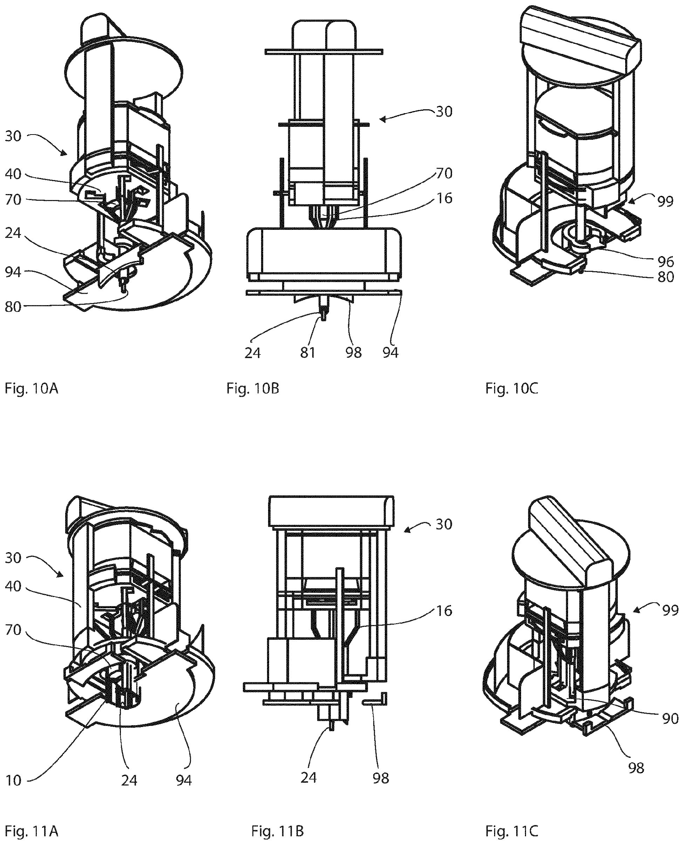

Figures 8A,B,C-11A,B,C illustrate together an embodiment of the tracheotomy apparatus 1 where a knife assembly 30 is transferred from the first state to the second state. Each of the figures shows three different perspective views of the tracheotomy apparatus 1.

Figures 8 A-C show an embodiment of the tracheotomy apparatus 1 prior to being in contact with a tissue. The knife assembly 30 is in the first state. The elongated member 81 is in primary position. The separator 70 is in a first position.

Figures 9 A-C illustrate the state of the tracheotomy apparatus 1 when in use, specifically when the tracheotomy apparatus 1 is positioned at the throat of a patient and a knife 10 is penetrating the tissue. The figure illustrates that the knife 10 extends beyond a targeting plate 98. Here, the elongated member 81 has been pushed into secondary position by the tissue. The knife assembly 30 is in the first state. The elongated member 81 is in secondary position. The separator 70 is in a first position.

Figures 10 A-C illustrate the tracheotomy apparatus 1 when in use, specifically when the tracheotomy apparatus 1 is positioned at the throat of the patient, the knife 10 has penetrated the tissue and the cutting edge 24 has passed into inner spacing of trachea. Here, the elongated member 81 is transferred back into the primary position by absence of the pressure from the tissue. The knife assembly 30 is in the first state. The elongated member 81 is in primary position. The separator 70 is in a first position. The base structure 94 is in more distal position compared to its position in Figures 9 A-B; the position transfer is caused by means of the spring mechanism 99.

Figures 11 A-C illustrate the tracheotomy apparatus 1 when in use, specifically when the tracheotomy apparatus 1 is positioned at the throat of the patient, the knife 10 has penetrated the tissue, the cutting edge 24 has passed into inner spacing of trachea, and a dilation of stoma is performed. The knife assembly 30 is transferred into the second state. The separator 70 is transferred from the first position to the second position. As the separator 70 is in this embodiment attached to a support base 90 of the knife support 40, the support base 90 is moved in the distal direction compared to its position in Figure 10. Figures 11 A-C illustrate that the knife 10 is thereby expanded, such that knife members 16 are positioned apart from each other. The mantle 22 is ruptured. Further, Figures 11 A-C illustrate that the targeting plate 98 is released from the base structure 94. The knife assembly 30 is in the second state. The separator 70 is in the second position.

From this point, if the tracheotomy apparatus 1 is in desired position, the knife support may be removed, thereby providing an aperture between the knife members for receiving, in this embodiment, a disposable tracheostomy device (not shown), such as a tube, a needle, a surgical instrument, or a catheter. Thus, the use of a tracheostomy system is illustrated and described.

### Item list

- 1: tracheotomy apparatus
- 10: knife
- 12: proximal portion of a knife
- 13: intermediate portion of the knife
- 14: distal portion of a knife
- 16: knife member
- 18: clip
- 20: distal end of the distal portion of the knife
- 22: mantle
- 24: cutting edge
- 26: opening at the distal end
- 28: fastening protrusion of a knife member
- 29: exterior surface of a knife member
- 30: knife assembly
- 40: knife support
- 42: locking member
- 44: apertures of the locking member
- 46: manual interface
- 48: connecting member
- 50: opening mechanism
- 60: actuator
- 62: spring
- 64: spring holding arrangement
- 65: rotating part
- 66: arm of actuator
- 67: handle
- 70: separator
- 80: indicator
- 81: elongated member of the indicator
- 82: audible member
- 83: plate
- 84: distal portion of the elongated member
- 90: support base of the knife support
- 92: apertures of the support base
- 94: base structure
- 95: base aperture
- 96: additional knife support
- 97: casing support
- 98: targeting plate
- 99: spring mechanism
- 99a: steel blade
- 99b: blade spring
- 100: exterior casing
- 111: first portion of a knife member
- 112: second portion a knife member
- 113: third portion a knife member
- 121: means for fastening
- 131: first edge of aperture
- 132: second edge of aperture
- 133: holding section
- 134: release section

## Claims

1. A tracheotomy apparatus (1) comprising:
a knife assembly (30) configured to be in a first state or in a second state,
the knife assembly (30) comprising:
- a knife support (40), and
- a knife (10) knife supported by the knife support, the knife having
a proximal (12) portion and a distal (14) portion, wherein the distal portion (14) is elongated, and has a distal (20) end, and extends from the knife support (40) in a distal direction,
wherein
the proximal portion (12) is attached to the knife support (40), the distal portion (14) comprises a cutting edge (24) at the distal end in the first state, and an opening (26) at the distal end in the second state, and is configured to receive a tracheostomy device extending through the opening (26) .

2. A tracheotomy apparatus (1) according to claim 1, further comprising an opening mechanism (50) comprising an actuator (60) operationally connected to the knife assembly, wherein the opening mechanism (50) is configured to transfer the knife assembly (30) from the first state to the second state.

3. A tracheotomy apparatus (1) according to any of the preceding claims, wherein the knife (10) comprises at least three elongated knife members (16), each knife member (16) forming part of the proximal portion (12) and the distal portion (14) of the knife.

4. A tracheotomy apparatus (1) according to claim 3, wherein the opening mechanism (50) further comprises a separator (70) operationally connected to the knife assembly (30) and to the actuator, and is configured to engage the knife (10) to transfer the knife assembly (30) from the first state to the second state.

5. A tracheotomy apparatus (1) according to any of the preceding claims, wherein the knife assembly (30) further comprises an indicator (80) configured to present a signal to a user at contact between the cutting edge (24) and a tissue during use, wherein the signal is an audible sound.

6. A tracheotomy apparatus (1) according to any of the claims 3-5, wherein:
the knife support (40) is configured to guide the knife members (16) transversely outwards in a transition from the first state to the second state.

7. A tracheotomy apparatus (1) according to any of the claims 3-6, wherein:
the knife support (40) comprises a support base (90) forming apertures (92), wherein each aperture (92) is configured to guide a single knife member (16) of the at least three knife members (16) at a transition from the first state to the second state of the knife assembly (30).

8. A tracheotomy apparatus (1) according to any of the claims 3-7, wherein:
each knife member (16) extends through respective aperture (92) of the support base (90), and comprises a fastening protrusion (28) preventing the knife member (16) from being released from the support base (90) in the first state and in a transition from the first state to the second state.

9. A tracheotomy apparatus (1) according to any of the claims 3-8, wherein:
the knife support (40) further comprises a locking member (42) having a blocking state in which it, for each knife member (16), blocks, or prevents passage of the fastening protrusion (28) through the respective aperture (92) of the support base (90) when the knife assembly (30) is in the first and the second state, and
the locking member (42) further has a releasing state in which, for each knife member (16), the locking member (42) allows passage the fastening protrusion (28) through the respective aperture (92) of the support base (90) when the knife assembly (30) is in the second state.

10. A tracheotomy apparatus (1) according to any of the claims 3-9, further comprising a base structure (94) located at the distal portion (14) of the knife (10), or at the distal end (20) of the distal portion (24) of the knife (10).

11. A tracheotomy apparatus (1) according to claim 10, further comprising a targeting plate attached to the base structure (94) and extending in the distal direction.

12. A tracheotomy apparatus (1) according any of the claims 10-11, wherein the base structure (94) comprises a spring mechanism (99) configured to releasably fix the base structure (94) to the knife support(40).

13. A tracheotomy apparatus (1) according any of claims 10-12, further comprising an exterior casing (100) releasably supporting the base structure (94) relative to the knife support(40).

14. A tracheotomy system according any of the preceding claims, wherein knife assembly (30) further comprises a flexible, or deformable, mantle flush with and extending along at least a portion of the distal portion of the knife (10) .

15. A tracheostomy system comprising a tracheotomy apparatus (1) according to any of the claims 1-14 and a tracheostomy device, such as a tube, a needle, a surgical instrument, or a catheter.
